# EUROPÄISCHE PATENTSCHRIFT

(11) **EP 2 415 738 B1**
(45) Veröffentlichungstag und Bekanntmachung des Hinweises auf die Patenterteilung: **02.12.2015**
(21) Anmeldenummer: 11175710.0
(22) Anmeldetag: 28.07.2011
(51) Int. Cl.: C07C 1/22, C07C 9/15

(54) **Verfahren zur Herstellung von linearen gesättigten Alkanen aus primären Alkoholen**
Method for producing linear saturated alkanes from primary alcohols
Procédé de fabrication d'alcanes saturés linéaires à partir d'alcools primaires

(30) Priorität: 05.08.2010 DE 102010033523
(43) Veröffentlichungstag der Anmeldung: 08.02.2012
(73) Patentinhaber: Saltigo GmbH, 51369 Leverkusen (DE)
(72) Erfinder: Mindach, Olaf, 51379 Leverkusen (DE); Kurth, Udo, 53909 Zülpich (DE); Lützenkirchen, Markus, 51373 Leverkusen (DE); Lange, Alexander, 51379 Leverkusen (DE)
(74) Vertreter: Pettrich, Klaus-Günter

(56) Entgegenhaltungen:
- WO-A1-2007/068371
- J. HENSEL, H. PINES: "Reactions of Alcohols V. Conversion of Alcohols to Ethers Over Nickel and Other Transition Metal Catalysts in the Presence of Hydrogen", JOURNAL OF CATALYSIS, Bd. 24, Nr. 2, 1. Februar 1972 (1972-02-01), Seiten 197-205, XP002661488, DOI: 10.1016/0021-9517(72)90062-0

## Beschreibung

Beschrieben wird ein Verfahren zur Herstellung von linearen gesättigten Alkanen aus primären Alkoholen durch Dehydroxymethylierung der primären Alkohole in Gegenwart eines Katalysators und Wasserstoff, wobei der Wasserstoffdruck im Bereich von 50 bis 150 bar und die Reaktionstemperatur im Bereich von 150 bis 250°C liegt und als Katalysator ein Ruthenium-Katalysator eingesetzt wird. Das Verfahren wird dabei als sogenanntes Sumpfverfahren durchgeführt.

Lineare gesättigte Alkane werden als Zusatzstoffe zur Herstellung von kosmetischen Zubereitungen wie beispielsweise Cremes, Gelen, Lotionen, Emulsionen, Wachse/Fette oder Pudern und Salben eingesetzt. Üblicherweise werden dabei lineare gesättigte ungradzahlige Alkane mit einer C-Zahl im Bereich von 7 bis 23 Kohlenstoffatomen verwendet. Beispiele für solche Verbindungen sind n-Nonan, n-Undekan, n-Tridekan oder n-Heptadekan. Üblicherweise werden diese primären linearen Alkane dabei auch als Gemisch, z. B. C₁₁/C₁₃-Alkangemisch in kosmetischen Formulierungen verwendet.

Hergestellt werden diese linearen gesättigten Alkane durch katalytische Dehydroxymethylierung von Fettalkoholen mit 8 bis 24 Kohlenstoffatomen nach dem folgenden Reaktionsschema:

R-CH₂-OH -> R-H + CH₄+ H₂O.

Der primäre Fettalkohol ist linear und weist üblicherweise eine geradzahlige Kohlenstoffatomzahl auf und besitzt somit in der Kohlenstoffkette ein Kohlenstoffatom mehr als das aus ihm hergestellte lineare Alkan.

Die Fettalkohole mit einer Kohlenstoffkettenlänge im Bereich von C₈-C₂₄ werden nach an sich bekannten Verfahren aus nachwachsenden Rohstoffen wie z. B. Kokos-, Palm- oder Palmkernöl über die Umesterung mit Methanol oder Ethanol und anschließender Hydrierung hergestellt.

Stand der Technik bei der Dehydroxymethylierung von Fettalkoholen ist die Umsetzung im Festbett an Nickel oder Nickel/Kupfer/Katalysatoren wie z. B. beschrieben in Helvetica Chimica Acta, Vol. 60, Fasc 8 (1977 - Nr. 290.)

Aus der WO 2007/068371 ist ein so genanntes Sumpfverfahren zur Herstellung von linearen gesättigten Alkanen aus Fettalkoholen bekannt, bei dem die Umsetzung ebenfalls an

Nickelkatalysatoren mit Wasserstoff bei Temperaturen um 250°C und Drücken zwischen 20 und 200 bar erfolgt. Nachteilig bei diesem Verfahren ist, dass das während der Umsetzung gebildete Wasser ständig aus der Reaktionsmischung entfernt werden muss, da es sonst die katalytische Dehydroxymethylierung nach diesem Verfahren beeinträchtigen würde.

Nach dem bisher bekannten Verfahren zur Herstellung von linearen gesättigten Alkanen aus Fettalkoholen werden Nickelkatalysatoren eingesetzt. Spuren des Nickelkatalysators können dabei in das Endprodukt gelangen, was bei deren Verwendung in kosmetischen Formulierungen in unerwünschter Weise zu allergischen Hautreaktionen führen könnte.

Aufgabe der vorliegenden Erfindung war es daher, ein neues Verfahren zur Herstellung von linearen gesättigten Alkanen aus der Dehydroxymethylierung von primären linearen Alkoholen wie z. B. Alkoholgemischen zur Verfügung zu stellen, bei dem das entstehende Reaktionswasser die katalytische Umsetzung des primären Alkohols zum linearen gesättigten Alkan nicht beeinträchtigt. Gefunden wurde nun ein Verfahren zur Herstellung von linearen gesättigten Alkanen, bei dem als Katalysator ein Ruthenium-Katalysator eingesetzt wird.

Gegenstand der Erfindung ist daher ein Verfahren zur Herstellung von linearen gesättigten Alkanen aus primären linearen Alkoholen durch Dehydroxymethylierung der primären Alkohole in Gegenwart eines Katalysators und Wasserstoff bei einem Druck im Bereich von 50 bis 150 bar und einer Temperatur im Bereich zwischen 150 und 250°C, bei dem der Katalysator ein Ruthenium-Katalysator ist. Ruthenium-Katalysatoren wurden bisher hauptsächlich für die Reduktion von Aldehyden und Ketonen eingesetzt.

Deren Verwendung zur katalytischen Dehydroxymethylierung von primären Alkoholen zu linearen gesättigten Alkanen wurde bisher noch nicht beschrieben. Beim erfindungsgemäßen Einsatz dieser Katalysatoren wird die katalytische Dehydroxymethylierung durch das während dieser Reaktion entstehende Reaktionswasser nicht beeinträchtigt, sodass nach dem erfindungsgemäßen Verfahren in vorteilhafter Weise die ständige Entfernung des Reaktionswassers aus dem Reaktionssystem entfallen kann. Im Vergleich zu den bisherigen Verfahren nach dem Stand der Technik, bei dem immer Nickelkatalysatoren eingesetzt wurden, ist das erfindungsgemäße Verfahren somit nicht nur energieeffizienter, sondern auch einfacher durchzuführen.

Das erfindungsgemäße Verfahren wird dabei als sogenanntes Sumpfverfahren durchgeführt, d. h., dass der Katalysator, gegebenenfalls suspendiert in einem Lösungsmittel, in einem rührbaren Druckbehälter (z.B. Autoklaven) vorgelegt wird und dann in diesen Druckbehälter zuerst das Wasserstoffgas bei dem angegebenen Druck und der angegebenen Temperatur und dann der primäre Alkohol bzw. das primäre Alkoholgemisch eingeleitet wird.

Als Katalysatoren sind geträgerte sowie ungeträgerte Ruthenium-Katalysatoren geeignet. Bevorzugt sind dabei geträgerte Ruthenium-Katalysatoren, insbesondere auf Kohle geträgerte Ruthenium-Katalysatoren. Ein Beispiel für einen derartigen Katalysator ist ein auf Kohle geträgerter Ruthenium-Katalysator mit 5 Gew.-% Ruthenium (z. B. kommerziell erhältlich unter der Bezeichnung K-0401^{®} von der Firma Hereaus).

Der Katalysator wird üblicherweise in einer Menge von 0,1 bis 10 Gew.-% bezogen auf die Menge an primären Fettalkoholen, eingesetzt. Bevorzugt beträgt die Menge an Katalysator dabei 0,5 bis 7 Gew.-%, besonders bevorzugt 1 bis 5 Gew.-%.

Die Temperatur, bei der das erfindungsgemäße Verfahren durchgeführt wird, liegt im Bereich von 150 bis 250°C bevorzugt im Bereich von 175 bis 225°C, besonders bevorzugt im Bereich von 185 bis 215°C.

Die Reaktionsdauer liegt im Bereich von 15 bis 180 min.

Als lineare Alkohole werden Fettalkohole der Formel R-OH eingesetzt, in der R für ein geradkettigen gesättigten linearen Alkylrest mit 8 bis 24 Kohlenstoffatomen, vorzugsweise 8 bis 18 Kohlenstoffatomen, insbesondere 12 bis 16 Kohlenstoffatomen steht. Neben den reinen Fettalkoholen können dabei auch Alkoholgemische von Alkoholen mit unterschiedlicher geradzahliger Kohlenstoffanzahl eingesetzt werden. Ein Beispiel für ein derartiges Alkoholgemisch ist z. B. ein Gemisch aus einem Alkohol mit 12 Kohlenstoffatomen und einem Alkohol mit 14 Kohlenstoffatomen. Derartige Alkoholgemische sind kommerziell erhältlich.

Nach dem erfindungsgemäßen Verfahren sind die ansonsten nach anderen Verfahren schwer zugänglich ungeradzahligen Alkane leicht erhältlich, z. B. ein C₁₁/C₁₃-Alkangemisch aus einem C₁₂-C₁₄-Alkoholgemisch.

Es hat sich als vorteilhaft erwiesen, den Katalysator im Gemisch mit dem gewünschten Endprodukt als Lösungsmittel (alternativ kann auch ein anderes Lösungsmittel benutzt werden) vorzulegen und unter den angegebenen Druck - bzw. Temperaturbedingungen das Wasserstoffgas zuzugeben und den primären Alkohol zuzupumpen, die Reaktion bei den angegebenen Reaktionsbedingungen für eine Dauer von 15 bis 180 min ablaufen zu lassen und anschließend das Reaktionsgemisch noch einige Zeit bei niedrigerer Temperatur (z. B. 150-200°C) nachzurühren. Der Katalysator wird anschließend von dem das Endprodukt enthaltenden Reaktionsgemisch abfiltriert. Der abfiltrierte Katalysator kann dann wieder im Gemisch mit etwas Endprodukt für eine weitere Reaktion eingesetzt werden. Durch eine Phasentrennung (Wasser/Alkan) des vom Katalysator abgetrennten Reaktionsgemisches erhält man dann das reine Alkan bzw. Alkangemisch. Die Ausbeuten an primären linearen Alkanen mit ungradzahliger Kettenlänge liegen dabei beim erfindungsgemäßen Verfahren bei über 95%. Gewünschtenfalls kann noch eine weitere destillative Aufarbeitung des erhaltenen primären linearen Alkans bzw. Alkangemisches erfolgen.

Die nachfolgenden Beispiele sollen die Erfindung weiter erläutern, ohne sie jedoch in ihrem Umfang zu begrenzen.

### Beispiele

### Beispiel 1:

Umsetzung eines Fettalkoholgemisches mit einer Kettenlänge von hauptsächlich C₁₂ und C₁₄ (Spektrum C10-C18):
Es wurden 400g C₁₁/C₁₃ Alkangemisch und 100g eines wasserfeuchten 5%Ru/C-Katalysators (K-0401^{®} der Firma Heraeus (50% Wasser)) in einem rührbaren Druckbehälter vorgelegt und unter 50 bar Wasserstoff auf 215°C aufgeheizt. Bei Erreichen der Temperatur wurde der Druck auf 150 bar H₂ gesteigert und es wurde innerhalb von 127 min 1000g eines C₁₂-C₁₄-Alkoholgemisches (Lorol C12-14S^{®} (Fa. Cognis GmbH) zugepumpt. Nach dem Zupumpen wurde die Temperatur auf 200°C gesenkt und noch insgesamt 75 min gerührt. Danach wurde das Reaktionsgemisch abgekühlt, entspannt und der Katalysator abfiltriert. Es ergab sich eine Auswaage von 1310g.

Eine GC-Analyse ergab folgende Zusammensetzung:

| | |
|---|---|
| 99,1% | Summe Kohlenwasserstoffe C₉-C₁₈ davon 95,9% mit ungradzahliger Kettenlänge |
| <0,1% | Summe Fettalkohole |
| <0,1% | Dimere Reaktionsprodukte |

Man erhielt ein farbloses, dünnflüssiges, geruchsarmes Produkt.

### Beispiel 2:

Es wurden 400g eines C11/C13-Alkangemisches und 93g des Katalysators wie in Beispiel 1 genannt, in einem rührbaren Druckbehälter vorgelegt und unter 50 bar Wasserstoff auf 215°C aufgeheizt. Bei Erreichen der Temperatur wurde der Druck auf 150 bar H₂ gesteigert und es wurde innerhalb von 125 min 1000g eines C₁₂/C₁₄-Alkoholgemisches (Lorol C12-14S^{®}, Fa. Cognis GmbH) zugepumpt. Nach dem Zupumpen wurde die Temperatur auf 200°C gesenkt und noch insgesamt 76 min gerührt. Danach wurde das Reaktionsgemisch abgekühlt, entspannt und der Katalysator abfiltriert. Es ergab sich eine Auswaage von 1325g.

Eine GC-Analyse ergab folgende Zusammensetzung:

| | |
|---|---|
| 99,0% | Summe Kohlenwasserstoffe C₉-C₁₈ davon 95,5% mit ungradzahliger Kettenlänge |
| <0,1% | Summe Fettalkohole |
| <0,1% | Dimere Reaktionsprodukte |

Man erhielt ein farbloses, dünnflüssiges, geruchsarmes Produkt.

## Patentansprüche

1. Verfahren zur Herstellung von linearen gesättigten Alkanen aus primären linearen Alkoholen der Formel R-OH, in der R für einen geradkettigen gesättigten linearen Alkylrest mit 8 bis 24 Kohlenstoffatomen steht, durch Dehydroxymethylierung der primären Alkohole in Gegenwart eines Katalysators und Wasserstoff bei einem Druck im Bereich von 50 bis 150 bar und einer Temperatur im Bereich von 150 bis 250°C, wobei der Katalysator, gegebenenfalls suspendiert in einem Lösungsmittel, in einem rührbaren Druckbehälter vorgelegt wird und dann in diesen Druckbehälter zuerst das Wasserstoffgas bei dem angegebenen Druck und der angegebenen Temperatur und dann der primäre Alkohol bzw. das primäre Alkoholgemisch eingeleitet wird, **dadurch gekennzeichnet, dass** der Katalysator ein Ruthenium-Katalysator ist und das bei der Dehydroxymethylierung der primären Alkohole gebildete Wasser während der Reaktion nicht aus dem Reaktionsgemisch entfernt wird.

2. Verfahren nach Anspruch 1, **dadurch gekennzeichnet, dass** der Katalysator in einer Menge im Bereich von 0,1 bis 10 Gew.-%, bezogen auf die Menge an primären Alkohol, eingesetzt wird.

3. Verfahren nach einem der Ansprüche 1 bis 2, **dadurch gekennzeichnet, dass** der Katalysator ein auf Kohle geträgerter Ruthenium-Katalysator ist.

4. Verfahren nach einem der Ansprüche 1 bis 3, **dadurch gekennzeichnet, dass** der primäre Alkohol ein Alkoholgemisch von Alkoholen mit unterschiedlicher geradzahliger Kohlenstoffanzahl ist.

5. Verfahren nach einen der Ansprüche 1 bis 4, **dadurch gekennzeichnet, dass** die Temperatur im Bereich von 175 bis 225°C liegt.

6. Verfahren nach einen der Ansprüche 1 bis 5, **dadurch gekennzeichnet, dass** nach Abschluss der Dehydroxymethylierung der Katalysator vom Reaktionsgemisch abgetrennt und gegebenenfalls in einem Gemisch mit dem entstandenen Alkan in einer weiteren Reaktion wieder eingesetzt wird.

## Claims

1. Process for producing linear, saturated alkanes from primary linear alcohols of formula R-OH, where R represents a straight-chain saturated linear alkyl radical having 8 to 24 carbon atoms, by dehydroxymethylation of the primary alcohols in the presence of a catalyst and hydrogen at a pressure in the range from 50 to 150 bar and a temperature in the range from 150°C to 250°C comprising initially charging the catalyst, optionally suspended in a solvent, into a stirrable pressure vessel and then introducing into said pressure vessel first the hydrogen gas at the stated pressure and the stated temperature and then the primary alcohol/the primary alcohol mixture, **characterized in that** the catalyst is a ruthenium catalyst and the water formed on dehydroxymethylation of the primary alcohols is not removed from the reaction mixture during the reaction.

2. Process according to Claim 1, **characterized in that** the catalyst is employed in an amount in the range from 0.1 to 10 wt% based on the amount of primary alcohol.

3. Process according to either of Claims 1 and 2, **characterized in that** the catalyst is a carbon-supported ruthenium catalyst.

4. Process according to any of Claims 1 to 3, **characterized in that** the primary alcohol is an alcohol mixture of alcohols having different, even numbers of carbon atoms.

5. Process according to any of Claims 1 to 4, **characterized in that** the temperature is in the range from 175°C to 225°C.

6. Process according to any of Claims 1 to 5, **characterized in that** it comprises removing the dehydroxymethylation catalyst from the reaction mixture after completion of the reaction and optionally reusing said catalyst in a mixture with the obtained alkane in a further reaction.

## Revendications

1. Procédé de fabrication d'alcanes saturés linéaires à partir d'alcools linéaires primaires de formule R-OH, dans laquelle R représente un radical alkyle linéaire saturé à chaîne droite de 8 à 24 atomes de carbone, par déshydroxyméthylation des alcools primaires en présence d'un catalyseur et d'hydrogène à une pression dans la plage allant de 50 à 150 bar et à une température dans la plage allant de 150 à 250 °C, le catalyseur, éventuellement suspendu dans un solvant, étant chargé dans un contenant sous pression agitable, puis tout d'abord l'hydrogène gazeux à la pression donnée et à la température donnée, puis l'alcool primaire ou le mélange d'alcools primaires étant introduits dans ce contenant sous pression, **caractérisé en ce que** le catalyseur est un catalyseur de ruthénium et l'eau formée lors de la déshydroxyméthylation des alcools primaires n'est pas éliminée du mélange réactionnel pendant la réaction.

2. Procédé selon la revendication 1, **caractérisé en ce que** le catalyseur est utilisé en une quantité dans la plage allant de 0,1 à 10 % en poids, par rapport à la quantité d'alcool primaire.

3. Procédé selon l'une quelconque des revendications 1 à 2, **caractérisé en ce que** le catalyseur est un catalyseur de ruthénium supporté sur du charbon.

4. Procédé selon l'une quelconque des revendications 1 à 3, **caractérisé en ce que** l'alcool primaire est un mélange d'alcools contenant différents nombres de carbones dans la chaîne droite.

5. Procédé selon l'une quelconque des revendications 1 à 4, **caractérisé en ce que** la température se situe dans la plage allant de 175 à 225 °C.

6. Procédé selon l'une quelconque des revendications 1 à 5, **caractérisé en ce que** le catalyseur est séparé du mélange réactionnel après la fin de la déshydroxyméthylation et éventuellement réutilisé dans une réaction ultérieure dans un mélange avec l'alcane formé.
